(19) Europäisches Patentamt
European Patent Office
Office européen des brevets

(11) **EP 4 668 197 A1**

(12) **EUROPEAN PATENT APPLICATION**

(43) Date of publication:
**24.12.2025 Bulletin 2025/52**

(21) Application number: **25183058.4**

(22) Date of filing: **16.06.2025**

(51) International Patent Classification (IPC):
$G06Q\ 50/02^{(2024.01)}$ $\quad G01N\ 33/24^{(2006.01)}$
$G06N\ 3/08^{(2023.01)}$ $\quad A01B\ 79/00^{(2006.01)}$

(52) Cooperative Patent Classification (CPC):
**A01B 79/005; G01N 33/24; G06N 3/045;
G06N 3/0464; G06N 3/08; G06N 3/084; G06N 3/09;
G06N 20/00; G06Q 50/02**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **18.06.2024 IN 202421046889**

(71) Applicant: **Tata Consultancy Services Limited
Maharashtra (IN)**

(72) Inventors:
• **MOHITE, Jayantrao**
**400601 Thane (West), Maharashtra (IN)**
• **SAWANT, Suryakant Ashok**
**411057 Pune, Maharashtra (IN)**
• **PANDIT, Ankur**
**453112 Indore, Madhya Pradesh (IN)**
• **PAPPULA, Srinivasu**
**500034 Hyderabad, Telangana (IN)**

(74) Representative: **Goddar, Heinz J.
Boehmert & Boehmert
Anwaltspartnerschaft mbB
Pettenkoferstrasse 22
80336 München (DE)**

(54) **METHOD AND SYSTEM FOR DYNAMIC ESTIMATION OF VERTICAL DISTRIBUTION OF SOIL ORGANIC CARBON (SOC)**

(57) This disclosure relates generally to a method and system for dynamic estimation of vertical soil organic carbon (SOC) at a region of interest (ROI). State-of-the-art methods are greatly dependent on satellite data. The satellite measurements are reliable for estimating the surface SOC, but it lacks accuracy when it comes to making estimations at sub-surface layers. The present disclosure addresses these problems through a method of dynamic estimation of vertical SOC by combining a domain-aware machine learning (ML) model and a process-based model. The domain-aware ML model incorporates spectral data, management practice data, soil spectral library (SSL) data, and a correction from the process-based model a corrected surface SOC. The process-based model further estimate depth-specific SOC fractions, along with fractions derived from global soil datasets and the SSL. By combining the domain-aware ML model and the depth-specific SOC fractions vertical SOC is estimated.

FIG. 2

## Description

CROSS-REFERENCE TO RELATED APPLICATIONS AND PRIORITY

The present application claims priority from Indian patent application no. 202421046889, filed on June 18, 2024.

TECHNICAL FIELD

The disclosure herein generally relates to estimation of Soil Organic Carbon (SOC) and, more particularly, to systems and methods for dynamic estimation of vertical SOC using a machine-learning model.

BACKGROUND

Soil Organic Carbon (SOC) is a critical component of soil health and fertility, representing the organic matter derived from plant and animal residues in the soil. It plays a pivotal role in supporting soil structure, water retention, and nutrient cycling. SOC is a key indicator of soil quality and is essential for sustaining agricultural productivity and ecosystem health. The SOC is crucial for how ecosystems function, influencing soil structure, moisture retention, and microbial activity. A precise estimation of SOC is vital for understanding and managing the health of natural ecosystems. In agriculture, SOC plays a direct role in soil fertility, impacting nutrient management, crop yield, and the environmental impact of farming practices. Accurate SOC estimation serves as a guide for farmers to optimize crop management practices to achieve sustainability. SOC is a major player in the global carbon cycle, contributing to carbon sequestration and efforts to mitigate climate change. Precise estimation is essential for understanding how soils store carbon, making SOC a key component in global climate change efforts. Monitoring SOC levels provide valuable insights into land-use changes, deforestation, and human-induced alterations, supporting sustainable land management and biodiversity conservation. It serves as a crucial tool for environmental monitoring, assists in making informed decisions about our changing landscapes. Therefore, precise SOC estimation is a critical part for managing ecosystems and addressing global environmental challenges. There are various techniques and approaches that have been developed for the SOC estimation such as laboratory-based methods, use of spectroscopy (hyperspectral sensing), use of satellite remote sensing, machine learning based modeling and geostatistical techniques such as spatial interpolation. However each of these methods have certain limitations in the precise and scalable estimation of the SOC. E.g. the laboratory analysis, like walkley-black titration and combustion methods are time-consuming, cumbersome and expensive. This makes them less suitable for large-scale spatial assessments, especially when frequent measurements are required. These methods often provide point-based measurements, limiting their ability to capture the spatial variability required for the comprehensive soil mapping. The geostatistical methods assume that the spatial variability remains constant over the study area (i.e. stationarity). This assumption may not hold in highly heterogeneous landscapes, impacting the accuracy of the predictions. Moreover, effective implementation often requires additional spatial covariates, and obtaining high-quality covariate data can be challenging, particularly for large regions. The spectroscopy methods such as visible-near-infrared (VNIR) and infrared (IR), present a non-destructive alternative for the rapid SOC assessment. However, they are mainly limited by the cost associated with data and time-needed for data collection which also limits the spatial scalability. Recent practices of the SOC estimation are based on machine learning (ML) that utilizes remote sensing data. However, conventional ML models often fail to predict the SOC in case of seasonal and regional variability. Moreover, conventional ML models can only provide SOC estimate at a surface or little deeper, at max up to 10 cm. This is due to limited penetration capabilities of the satellite-based earth observations.

SUMMARY

Embodiments of the present disclosure present technological improvements as solutions to one or more of the above-mentioned technical problems recognized by the inventors in conventional systems. For example, in one embodiment, a method for dynamic estimation of vertical SOC by a ML model for the region of interest is provided. The method includes receiving, via one or more hardware processors, (a) a plurality of spectral embeddings received from satellite data, (b) a plurality of temporal embeddings derived from management proxies, and (c) an attention score from a soil spectral library (SSL) based on one or more soil spectral signatures derived from the SSL for a region of interest (ROI). The plurality of spectral embeddings transforms high-dimensional spectral information received from the satellite data into a dense vector space. This enables subsequent layers of the ML model to efficiently extract and learn relevant patterns. The plurality of temporal embeddings captures temporal dynamics of agricultural management practices to enable the ML model to adapt evolving agricultural scenarios. The soil spectral signatures are utilized to embed an attention mechanism in the ML model wherein the attention mechanism trains the ML model to adapt characteristic variations among soil and generates an attention score. The method further includes integrating, via one or more hardware processors, the plurality of spectral embeddings, the plurality of temporal embeddings, and the attention score into a joint feature space to obtain a domain-aware machine learning (ML) model. The domain-aware ML model estimates surface SOC in various stages and also acquires the SOC information from diverse data sources to consider current as well as historical SOC in the ROI. The method further includes applying, via one or more hardware processors, a loss function to the domain-aware ML model wherein the loss function employs the plurality of temporal embeddings, and the soil spectral signatures to minimize an error in the SOC prediction for the ROI. This incorporates spectral soil library (SSL)-based information and management proxies and serves to guide the domain-aware ML model during training. The method further includes generating, via one or more hardware processors, a temporal SOC for the ROI by a process-based model wherein the process-based model utilizes a plurality of factors affecting a historical surface SOC and a historical vertical SOC over a period to predict the temporal SOC. The temporal SOC estimation by a process-based model involves identifying the historical surface SOC and the historical vertical SOC from the spectral embeddings, the temporal embeddings and the SSL data. The temporal SOC includes both surface SOC and vertical SOC dynamics over a period of time. The method further includes obtaining, via one or more hardware processors, a correction factor by subtracting the temporal SOC predicted by the process-based model and the surface SOC estimated from the domain-aware ML model. The correction factor captures the nuanced discrepancies between the domain-aware ML model and the process-based model. The correction factor is then applied to the surface SOC estimates from the domain-aware ML model. The method further includes estimating, via one or more hardware processors, a corrected surface SOC by applying the correction factor to the surface SOC estimates obtained from the domain-aware ML model. The correction factor serves as an adjustment mechanism to align the ML-based estimations with the dynamics simulated by the process-based model. The method further includes obtaining, via one or more hardware processors, one or more depth specific SOC fractions of the ROI. The domain-aware ML model receives a static vertical SOC profile at a plurality of depths from a global soil information system. A temporally weighted SOC profile is generated by the process-based model, wherein the process-based model calculates the temporal SOC at the plurality of depths. Further, a plurality of SOC fractions are generated at the plurality of depths from the SSL, wherein the plurality of SOC fractions represents one or more general trends of one or more soil properties in the ROI. Then, the static vertical SOC fraction received from the global soil information system, the temporally weighted SOC derived from the process-based model, and the plurality of SOC fractions estimated from the SSL are combined to obtain the one or more depth specific SOC fractions. The method further includes estimating, via one or more hardware processors, a vertically distributed SOC for the ROI by combining the corrected surface SOC and the one or more depth specific SOC fractions at the ROI.

In another aspect, a system for dynamic estimation of vertical SOC is provided. The system includes at least one memory storing programmed instructions; one or more Input /Output (I/O) interfaces; and one or more hardware processors, vertical SOC estimation model, domain aware ML module process-based module, SSL data processing module, and GSI data processing module, operatively coupled to a corresponding at least one memory, wherein the system is configured to receive, via one or more hardware processors, (a) a plurality of spectral embeddings received from satellite data, (b) a plurality of temporal embeddings derived from management proxies, and (c) an attention score from a soil spectral library (SSL) based on one or more soil spectral signatures derived from the SSL for a region of interest (ROI). The plurality of spectral embeddings transforms high-dimensional spectral information received from the satellite data into a dense vector space. This enables subsequent layers of the ML model to efficiently extract and learn relevant patterns. The plurality of temporal embeddings capturing temporal dynamics of agricultural management practices enable the ML model to adapt evolving agricultural scenarios. The soil spectral signatures are utilized to embed an attention mechanism in the ML model wherein the attention mechanism trains the model to adapt characteristic variations among soil and generates an attention score. The system is configured to integrate, via one or more hardware processors, the plurality of spectral embeddings, the plurality of temporal embeddings, and the SSL attention score into a joint feature space to obtain a domain-aware machine learning (ML) model. The domain-aware ML model estimates surface SOC in various stages and also acquires the SOC information from diverse data sources to consider current as well as historical SOC of the ROI. The system is configured to apply, via one or more hardware processors, a loss function to the domain-aware ML model wherein the loss function employs the plurality of temporal embeddings, and the soil spectral signatures to minimize an error in the SOC prediction for the ROI. The model incorporates SSL based information and management proxies and serves to guide the ML model during training. The system is configured to generate, via one or more hardware processors, a temporal SOC for the ROI by a process-based model wherein the process-based model utilizes a plurality of factors affecting a historical surface SOC and a historical vertical SOC over a period of time to predict the temporal SOC. The temporal SOC includes both surface SOC and vertical SOC dynamics over a period of time. The system is configured to obtain, via one or more hardware processors, a correction factor by subtracting the temporal SOC predicted by the process-based model and the surface SOC estimated from the domain-aware ML model. The correction factor captures the nuanced discrepancies between the domain-aware model and process-based model. The correction factor is then applied to the surface SOC estimates from the domain-aware ML model. The system is configured to estimate, via one or more hardware processors, a corrected surface SOC by applying the correction factor to the surface SOC estimates obtained from the domain-aware ML model. The correction factor serves as an adjustment mechanism to align the ML-based estimations with the dynamics simulated by the process-based model. The system is configured to obtain one or more depth specific SOC fractions of the ROI. The domain-aware ML model receives a static vertical SOC profile at a plurality of depths from a global soil information system. Then, a temporally weighted SOC profile is generated by the process-based model, wherein the process-based model calculates the temporal SOC at the plurality of depths. Further, a plurality of SOC fractions are generated at the plurality of depths from the SSL, wherein the plurality of SOC fractions represents one or more general trends of one or more soil properties in the ROI. Then, the static vertical SOC fraction received from the global soil information system, the temporally weighted SOC derived from the process-based model, and the plurality of SOC fractions estimated from the SSL are combined to obtain the one or more depth specific SOC fractions. The system is configured to estimate a vertically distributed SOC for the ROI by combining the corrected surface SOC and the one or more depth specific SOC fractions at the ROI.

In yet another aspect, a computer program product including a non-transitory computer-readable medium embodied therein a computer program for dynamic estimation of vertical SOC is provided. The computer readable program, when executed on a computing device, causes the computing device to receive (a) a plurality of spectral embeddings received from satellite data, (b) a plurality of temporal embeddings derived from management proxies, and (c) an attention score from a soil spectral library (SSL) based on one or more soil spectral signatures derived from the SSL for a region of interest (ROI). The plurality of spectral embeddings transforms high-dimensional spectral information received from the satellite data into a dense vector space. This enables subsequent layers of the ML model to efficiently extract and learn relevant patterns. The plurality of temporal embeddings capturing temporal dynamics of the agricultural management practices to enable the ML model to adapt evolving agricultural scenarios. The soil spectral signatures are utilized to embed an attention mechanism in the ML model wherein the attention mechanism trains the model to adapt characteristic variations among soil and generates an attention score. The computer readable program, when executed on a computing device, causes the computing device to integrate the plurality of spectral embeddings, the plurality of temporal embeddings, and the SSL attention score into a joint feature space to obtain a domain-aware machine learning (ML) model. The domain-aware ML model estimates surface SOC in various stages and also acquires the SOC information from diverse data sources to consider current as well as historical SOC of the ROI. The computer readable program, when executed on a computing device, causes the computing device to apply a loss function to the domain-aware ML model wherein the loss function employs the plurality of temporal embeddings, and the soil spectral signatures to minimize an error in the SOC prediction for the ROI. The model incorporates SSL based information and management proxies and serves to guide the ML model during training. The computer readable program, when executed on a computing device, causes the computing device to generate a temporal SOC for the ROI by a process-based model wherein the process-based model utilizes a plurality of factors affecting a historical surface SOC and a historical vertical SOC over a period of time to predict the temporal SOC. Temporal SOC includes both surface SOC and vertical SOC dynamics over a period of time. The computer readable program, when executed on a computing device, causes the computing device to obtain a correction factor by subtracting the temporal SOC predicted by the process-based model and the surface SOC estimated from the domain-aware ML model. The correction factor captures the nuanced discrepancies between the domain-aware model and the process-based model. The correction factor is then applied to the surface SOC estimates from the domain-aware ML model. The computer readable program, when executed on a computing device, causes the computing device to estimate a corrected surface SOC by applying the correction factor to the surface SOC estimates obtained from the domain-aware ML model. The correction factor serves as an adjustment mechanism to align the domain-aware ML-based estimations with the dynamics simulated by the process-based model. The computer readable program, when executed on a computing device, causes the computing device to obtain one or more depth specific SOC fractions of the ROI. The domain-aware ML model receives a static vertical SOC profile at a plurality of depths from a global soil information system. A temporally weighted SOC profile is generated by the process-based model, wherein the process-based model calculates the temporal SOC at the plurality of depths. Further, a plurality of SOC fractions are generated at the plurality of depths respectively from the SSL, wherein the plurality of SOC fractions represents one or more general trends of one or more soil properties in the ROI. Then, the static vertical SOC fraction received from the global soil information system, the temporally weighted SOC derived from the process-based model, and the plurality of SOC fractions estimated from the SSL are combined to obtain the one or more depth specific SOC fractions. The computer readable program, when executed on a computing device, causes the computing device to estimate a vertically distributed SOC for the ROI by combining the corrected surface SOC and the one or more depth specific SOC fractions at the ROI.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates an exemplary block diagram of a system 100 for dynamic estimation of a vertical soil organic carbon (SOC), according to some embodiments of the present disclosure.

FIG. 2 illustrates an exemplary environment for dynamic estimation of the vertical SOC, according to some embodiments of the present disclosure.

FIG. 3 illustrates an overall framework for dynamic estimation of vertical SOC while processing satellited based data, soil library-based data and management practices-based data, according to some embodiments of the present disclosure.

FIGS. 4A and 4B depict flow diagrams of an illustrative method for the dynamic estimation of the vertical SOC, using the system of FIG. 1, according to some embodiments of the present disclosure.

FIG. 5 illustrates agricultural land division into vertical layers and spatial zones to estimate the vertical SOC in a region of interest (ROI), according to some embodiments of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

As used herein the term "soil organic carbon (SOC)" refers to an amount of carbon retained in the soil after the decomposition of the organic content.

As used herein the term "surface SOC" refers to concentration of an organic carbon in topmost layer of the soil profile, typically top 10 centimeters.

As used herein the term "vertical SOC" refers to amount of SOC present to a deeper layers of the soil, generally 10 cm and below.

As used herein the term "corrected surface SOC" refers to a predicted surface SOC value by a domain-aware ML model according to the present disclosure. The surface SOC is estimated to be using satellite data which is further corrected using the process-based model. The surface SOC estimated after correction is the corrected surface SOC.

As used herein the term "temporal SOC" refers to a SOC estimate obtained by analysing surface SOC and vertical SOC from a historical data collected from a spectral soil library (SSL). This includes variations in soil organic carbon levels over time. These changes can be influenced by various factors including climate conditions, land use practices, vegetation cover, and soil management techniques.

Soil is the largest pool of terrestrial organic carbon in the biosphere, storing more carbon than is contained in plants and the atmosphere combined. One aspect of the organic carbon pool that remains poorly understood is its vertical distribution in the soil and accompanying relationships with climate and vegetation. It is found that vegetation is a major determinant of the vertical distribution of the SOC. Although climate and soil texture are the primary regional controls of the total amount of SOC, their influence on the vertical distribution of the SOC may be eclipsed by the effects of plant allocation. Plant production and decomposition determines carbon inputs to the soil profile, and plant allocation above and below ground and between shallow and deep roots may leave distinct imprints on the relative distribution of soil carbon with depth. Machine learning is amongst the latest techniques to characterize the SOC vertical distribution using mathematical functions by extrapolating the surface SOC estimates to the deeper layers. However, the mapping of SOC variation with depth is difficult to achieve with accuracy and the prediction accuracy generally decreases with depth and the environmental covariates used may only explain the variation of SOC in the top 50 cm of the soil profile.

In the present disclosure, a method and a system for dynamic estimation of vertical distribution of the SOC is presented. The disclosed technical approach for the surface SOC estimation involves creating a domain-aware ML model that considers diverse set of data from satellite, management practices and soil spectral library (SSL). Satellite data is mainly utilized from data sources, including optical and Synthetic Aperture Radar (SAR) remote sensing which involves both raw bands and indices. Moreover, the surface SOC is significantly influenced by temporal management practices followed throughout the seasons, hence the present disclosure consider proxies associated with the management zones along with time information. Also, a plurality of input data from the SSL provides an information on spectral characteristics associated with various soil types and SOC levels at one or more depths in the region of interest (ROI) is utilized. The domain-aware ML model mainly considers spectral embeddings from a plurality of satellite data, temporal embeddings from a plurality of proxies derived from one or more management zones and an attention-based mechanism for the SSL derived features. Consideration of domain specific adaptations is utilized for spatio-temporal scalability. After estimating the surface SOC, a correction step utilizes the process-based models, like the RothC model, to correct the surface SOC. A relationship is established between the machine learning based and the process-based estimates, and the correction factor is generated which is applied to enhance accuracy. This approach provides a robust framework for the scalable, spatio-temporally aware, and physically realistic surface SOC estimation, essential for effective land management and environmental monitoring. Further, the surface SOC derived above is utilized as one of the input to estimate a vertical distribution of the SOC. Along with the derived surface SOC, the method also utilizes a plurality of depth informed spectral embeddings. Therefore, dynamic estimation of the vertical SOC is based on the data about the surface SOC obtained as above, the vertical SOC profile derived from the process-based model and the depth informed spectral embedding along with SSL. Initially, a plurality of zones representing uniformity in terms of cropping pattern, agro-climate and soil profile are created based on stratified random sampling. Temporally weighted SOC is estimated at each depth for each of the zones using the temporal SOC obtained from the process-based model. Moreover, the SOC fractions for each depth are determined based on the available soil type, texture and depth informed spectral embeddings derived from the SSL. Further, a static SOC fraction obtained from a global soil information system (Such as SoilGrids™) for each depth is also considered. Finally weighted average of the SOC fractions derived from the process-based models, depth informed spectral embeddings from the SSL and the SoilGrids™ data are combined to estimate the plurality of SOC fractions at each depth. These fractions are then used along with the corrected surface SOC to estimate the vertical SOC in the region of interest.

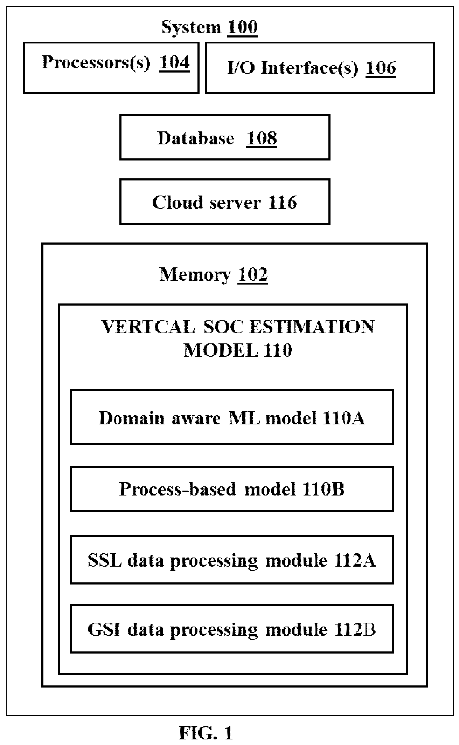

Referring now to the drawings, and more particularly to FIG. 1 through FIG. 5, where similar reference characters denote corresponding features consistently throughout the figures, there are shown preferred embodiments, and these embodiments are described in the context of the following exemplary system and/or method.

FIG. 1 illustrates an exemplary block diagram of a system 100 for dynamic estimation of vertical soil organic carbon (SOC), according to some embodiments of the present disclosure.

In an embodiment, the system 100 includes one or more processors 104, communication interface device(s) or input/output (I/O) interface(s) 106, and one or more data storage devices or memory 102 operatively coupled to the one or more processors 104. The one or more processors 104 that are hardware processors can be implemented as one or more microprocessors, microcomputers, microcontrollers, digital signal processors, central processing units, state machines, graphics controllers, logic circuitries, and/or any devices that manipulate signals based on operational instructions. Among other capabilities, the processor(s) are configured to fetch and execute computer-readable instructions stored in the memory. In the context of the present disclosure, the expressions 'processors' and 'hardware processors' may be used interchangeably. In an embodiment, system 100 can be implemented in a variety of computing systems, such as, laptop computers, notebooks, hand-held devices, workstations, mainframe computers, servers, a network cloud, and the like. The I/O interface (s) 106 may include a variety of software and hardware interfaces, for example, a web interface, a graphical user interface, and the like and can facilitate multiple communications within a wide variety of networks and protocol types, including wired networks, for example, LAN, cable, etc., and wireless networks, such as WLAN, cellular, or satellite. In an embodiment, the I/O interface(s) 106 can include one or more ports for connecting a number of devices such as the user terminals enabling user to communicate with system via the chat bot UI or enabling devices to connect with one another or to another server. The memory 102 may include any computer-readable medium known in the art including, for example, volatile memory, such as static random-access memory (SRAM) and dynamic random-access memory (DRAM), and/or non-volatile memory, such as read only memory (ROM), erasable programmable ROM, flash memories, hard disks, optical disks, and magnetic tapes. In an embodiment, memory 102 may include a database or repository. Memory 102 may comprise information pertaining to input(s)/output(s) of each step performed by the processor(s) 104 of the system 100 and methods of the present disclosure. In an embodiment, the database may be external (not shown) to the system 100 and coupled via the I/O interface 106. The memory 102 includes a vertical SOC estimation model 110, which further comprises of a domain-aware ML model 110A, a process-based model 110B, a Soil spectral library (SSL) data processing module 112A and a global soil information system (GSI) data processing modules data collection module 112 B. The domain-aware ML model 110A performs precise estimation of surface SOC by integrating diverse data sources. This includes optical and synthetic aperture radar (SAR) remote sensing indices, proxies derived from management practices, timestamp data, and spectral insights from the SSL. Through careful feature engineering, these datasets are harmonized to ensure consistent scaling through normalization and standardization. In an embodiment, the ML algorithm selected for model training is Random Forest regression. The model training involves capturing historical data to integrate the intricacies of the SOC dynamics. The process-based model 110B simulate the dynamics of SOC in the soil. The precision of SOC estimates is enhanced by the process-based modeling wherein a correction step is applied to the surface SOC estimated by the domain-aware ML model. A crucial aspect of this correction process involves establishing a relationship between the ML-based surface SOC estimates and those derived from the process-based models. Correction factor is then applied to the initial ML estimates, ensuring that the final SOC predictions align more closely with the mechanistic insights offered by the process-based models. This dual-phase approach, encompassing domain-aware ML modeling and correction using the process-based model provides a holistic and robust framework for advancing the accuracy and reliability of the surface SOC estimation, essential for effective land management and environmental monitoring. The SSL data processing module 112A receives a harmonized database consisting of multiple spectral libraries in both the visible-near infrared (VNIR) and mid infrared (MIR) regions of the electromagnetic spectrum with associated traditionally measured soil properties. The spectral libraries are then processed by the SSL data processing module 112A to derive meaningful inferences about various depth at a region of interest. SSL data processing module 112A derives SSL attention-based features from the SSL. The SSL inputs further improve spatial and temporal scalability of the vertical SOC estimation model 110. The GSI data processing module 112B processes spatial data received from the global soil information system. The GSI system compiles soil information collected by national institutions and other data holding entities. The GSI system provides a decentralized global soil data platform that is nationally/regionally federated and globally harmonized. The GSI data processing module 112B derives one or more static vertical SOC fractions at multiple depths (i.e. depth specific SOC fractions) for a region of interest. The memory 102 further includes a plurality of modules (not shown here) comprises programs or coded instructions that supplement applications or functions performed by the system 100 for executing different steps involved in the data rate prediction and prioritization. The plurality of modules, amongst other things, can include routines, programs, objects, components, and data structures, which perform particular tasks or implement particular abstract data types. The plurality of modules may also be used as, signal processor(s), node machine(s), logic circuitries, and/or any other device or component that manipulates signals based on operational instructions. Further, the plurality of modules can be used by hardware, by computer-readable instructions executed by one or more hardware processors 104, or by a combination thereof. The plurality of modules can include various sub-modules (not shown).

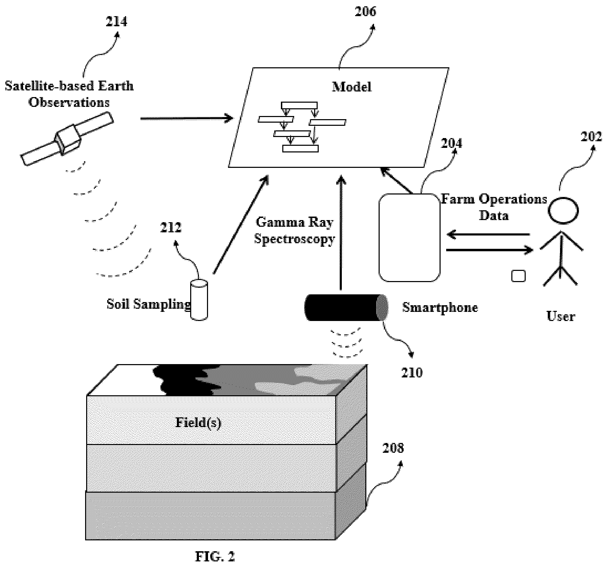

FIG. 2 illustrates an exemplary environment for dynamic estimation of vertical SOC, according to some embodiments of the present disclosure.

Referring to FIG. 2, there is shown a scenario wherein a user having a smartphone is interested in dynamic estimation of vertical SOC in his fields at a region of interest. Characterization of spatial variability in soil properties is crucial for users/farmers to reduce the risk of crop failure, to improve the efficiency of decision making and to benefit in both the economic and environmental sense. The vertical SOC estimation model 110 is deployed in the smartphone that receives inputs from the user. At the same time, the vertical SOC estimation model 110 gathers information from satellite-based earth observations as well as from various databases specific to soil related information including soil sampling and gamma ray spectroscopy databases. Based on the user inputs as well as the information from various databases, the model estimates vertical SOC at the region of interest. Considering the scenario the user 202 inputs farm operations data to the smartphone 204. The farm operations data is an exhaustive data about soil characteristics, crop patterns, farming practice, regional information, seasonal information and the like. The smartphone 204 has a vertical SOC estimation model 110 to estimate dynamic vertical SOC. The vertical SOC estimation model 110 is based on machine learning that receives a plurality of inputs and processes these inputs to estimate dynamic vertical SOC. The field 206 where the region of interest lies is monitored using the earth observation satellites for real-time information as well as historical data stored in various databases supporting the soil related information. The vertical SOC estimation model 110 further receives gamma-ray spectroscopy data 208. The gamma-ray spectroscopy is a relatively new approach in characterizing soil properties for arable farming and the focus has been to evaluate the technology in a soil mapping framework. It is ground-based proximal soil sensing methods that can provide information on soil properties at a high spatial resolution. Using gamma-ray spectroscopy, soil variables can be mapped at a high spatial resolution. Gamma rays can be related with clay mineralogy and soil chemistry and the concentration of radionuclides can be related with soil properties using simple correlation method. Furthermore, unlike EMI sensors, metal objects do not attenuate gamma rays while soil measurement. Further, the vertical SOC estimation model 110 receives soil sampling data 210. The soil sampling data 210 is obtained from global soil information system and soil spectral library (SSL). The global soil information system such as SoilGrids™ is a platform for digital soil mapping based on global compilation of soil profile data and environmental layers. The SoilGrids™ is based on state-of-the-art machine learning methods to map the spatial distribution of soil properties across the globe. The SSL is an open-source harmonized database consisting of multiple spectral libraries in both the visible-near infrared (VNIR) and mid infrared (MIR) regions of the electromagnetic spectrum with associated traditionally measured soil properties. The SSL facilitates information related to soil type, texture and depth informed spectral embeddings to the vertical SOC estimation model 110. The vertical SOC estimation model 110 further receives satellite data 212 (i.e. satellite based earth observations) including optical and Synthetic Aperture Radar (SAR) remote sensing which involves both raw bands and indices. The vertical SOC estimation model 110 utilizes spectral embeddings from the satellite data 214 while estimating the vertical SOC. Therefore, entire components integrates with each other and processed by the vertical SOC estimation model 110 to estimate the vertical SOC upon user request.

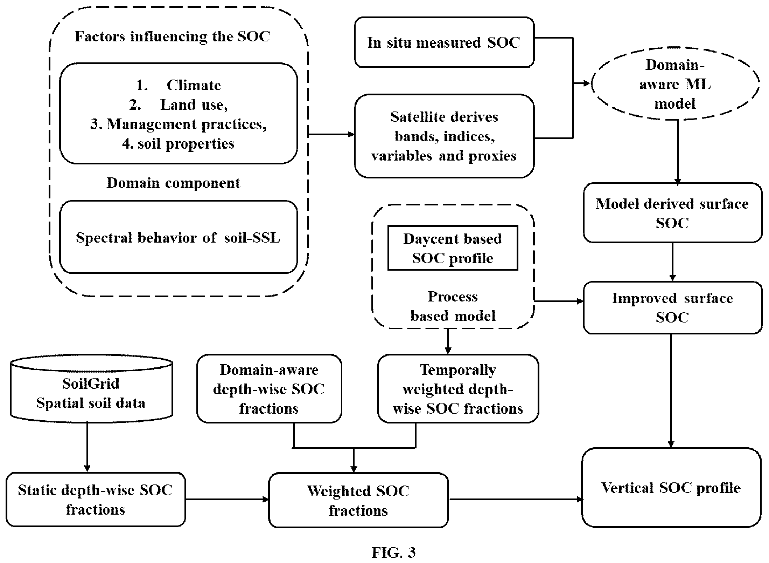

FIG. 3 illustrates an overall framework for dynamic estimation of vertical SOC while processing satellited based data, library-based data and management practices-based data, according to some embodiments of the present disclosure.

Referring to FIG. 3 illustrates an overall framework for dynamic estimation of vertical SOC while processing satellite data, SSL data and management practices data, according to some embodiments of the present disclosure. The dynamic estimation of vertical SOC involves an integration of the domain-aware ML model 110A, a process-based model 110B and an inferences received from various repositories and soil monitoring systems. In developing a domain-aware ML model 110A for precise estimation of surface soil organic carbon (SOC), diverse data sources are integrated. The domain-aware ML model 110A receives an *in-situ* measured SOC data 302 and satellite derived bands, indices, variables and proxies data 304. Through careful feature engineering, the received data is harmonized ensuring consistent scaling through normalization and standardization.

According to an embodiment of the present invention, the choice of a machine learning algorithm, such as random forest regression, is deliberate, and its training involves historical data to capture the intricacies of SOC dynamics. The model undergoes rigorous validation, assessing its adaptability across various geographic regions and time periods. Fine-tuning refines the model for enhanced scalability and spatio-temporal awareness, enabling accurate SOC estimates crucial for informed land management. The domain-aware ML model 110A utilizes random forest regression algorithm and the model training involves historical data to capture the intricacies of the SOC dynamics. The domain-aware ML model 110A undergoes rigorous validation, assessing its adaptability across various geographic regions and time periods. The satellite derived bands, indices, variables and proxies data 304 considers a plurality of factors influencing the SOC and are forms the domain components for training the domain-aware ML model 110A. Such factors comprise climate including temperature and rainfall, land use scenarios including tillage and cropping intensity, soil properties including texture, and roughness. Further, domain components involve spectral signatures derived from the SSL. The inclusion of an attention mechanism for the SSL features is to dynamically focus on relevant spectral signatures from the SSL. This mechanism allows the domain-aware ML model 110A to assign varying levels of importance to different SSL features, ensuring that the model emphasizes the most informative components. This helps the domain-aware ML model 110A to adapt its attention to different soil characteristics, enhancing overall performance.

A loss function is applied to the domain-aware ML model 110A wherein the loss function employs the plurality of temporal embeddings, and the soil spectral signatures to minimize an error in the SOC prediction for the ROI. This results in a model derived surface SOC 306. Further the process-based model 110B derives temporal SOC 308. The temporal SOC 308 for the ROI by a process-based model 110B is estimated wherein the process-based model 110B utilizes a plurality of factors affecting a historical surface SOC and a historical vertical SOC over a period to predict the temporal SOC. A DAYCENT model (i.e. daily time-step version of the CENTURY biogeochemical model) is utilized in deriving the temporal SOC from the process-based model 110B. Further, the precision of SOC estimates is enhanced by integrating insights from the process-based model 110B. This is done by a correction factor. A crucial aspect of this correction process involves establishing a relationship between the ML-based surface SOC estimates and those derived from the process-based model 110B. The correction factor is obtained by subtracting the temporal SOC 308 predicted by the process-based model 110B and the model derived surface SOC 306 estimated from the domain-aware ML model 110A.

Finally, a corrected surface SOC 310 is calculated by applying the correction factor to the surface SOC 306 estimates obtained from the domain-aware ML model 110A. Then spatial soil data is extracted from SoilGrids™ 312 to factor gridded soil property maps at different depths worldwide in the dynamic vertical SOC estimation. SoilGrids™ provides static dataset and serves as a baseline vertical SOC profile. The spatial soil data is further utilized to estimate fractions of SOC at various depths relative to the surface SOC for a given pixel/point. This results in estimation of static depth-wise SOC fractions 314. Once again, the process-based model 110B simulates the temporal and vertical profile of the SOC. This data is utilized to get the temporally weighted SOC profile 316 for each zone. Alongside, domain-aware depth wise SOC fractions 318 are obtained from the domain-aware model 110A. The temporally weighted SOC profile 316 and the domain-aware depth wise SOC fractions 318 are combined to generate weighted SOC fractions 320. Finally, the weighted SOC fractions 320 and the static fractions derived from SoilGrids™ 314 are combined to derive vertical SOC profile 322.

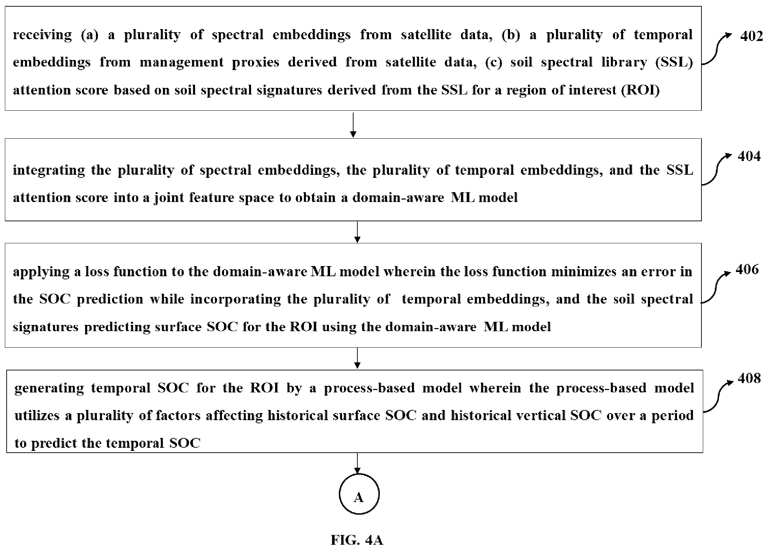

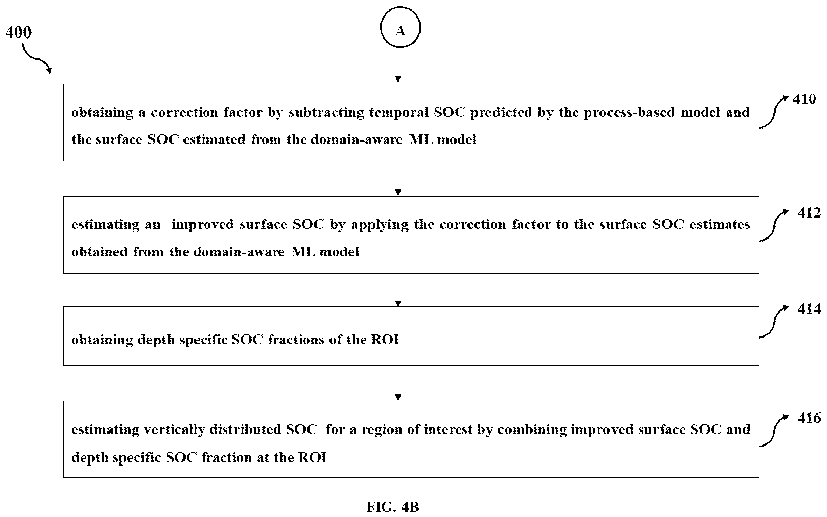

FIG. 4 is a flow diagram of an illustrative method 400 for dynamic estimation of vertical SOC, according to some embodiments of the present disclosure.

The steps of method 400 of the present disclosure will now be explained with reference to the components or blocks of the system 100 as depicted in FIG. 1 through FIG. 5. Although process steps, method steps, techniques or the like may be described in a sequential order, such processes, methods, and techniques may be configured to work in alternate orders. In other words, any sequence or order of steps that may be described does not necessarily indicate a requirement that the steps be performed in that order. The steps of processes described herein may be performed in any order practically. Further, some steps may be performed simultaneously. The vertical SOC estimation model 110 is a combination of domain-aware ML model 110A and process-based model 110B for precise estimation of the surface SOC wherein diverse data sources are integrated. This includes optical and SAR-based remote sensing indices, proxies derived from management practices, timestamp data, and spectral insights from a soil spectral library. Through careful feature engineering, these datasets are harmonized to ensure consistent scaling through normalization and standardization. In an embodiment of the present disclosure, the random forest regression is applied, and its training involves historical data to capture the intricacies of the SOC dynamics. The model undergoes rigorous validation, assessing its adaptability across various geographic regions and time periods. Fine-tuning refines the model for enhanced scalability and spatio-temporal awareness, enabling accurate SOC estimates crucial for informed land management.

At step 402 of the method 400, the one or more hardware processors 104 are configured to receive a plurality of spectral embeddings, a plurality of temporal embeddings and attention score based spectral soil library (SSL). The plurality of spectral embeddings are received from satellite the data. The satellite data is mainly utilized from data sources, including optical and Synthetic Aperture Radar (SAR) remote sensing which involves both raw bands and indices. The SAR image data are a 2D array of complex numbers with indices representing, for example, changing range and changing azimuth coordinates. Like signal data, each sample includes quantized amplitude and phase (or alternatively, in-phase and quadrature) components. Each element of the array represents an image pixel with amplitude related to the strength of the radar backscatter coefficient in the corresponding scene area. The plurality of temporal embeddings are derived from the management proxies. The surface SOC is significantly influenced by temporal management practices followed throughout the seasons. Hence, it is essential to factor proxies associated with those management zones along with time information. The management practices data is gathered in various formats covering crop practices, seasonal variation, regional specifics etc. The management practice data is then processed using custom scripts. Further, algorithms based on spatial interpolation are utilized in management practice data. And historical records of agricultural activities (e.g., planting, irrigation, harvesting) are also acquired from temporal analysis datasets. Further, a SSL attention score based on soil spectral signatures is derived from the SSL for a region of interest (ROI).

Additionally, SSL provides the information on spectral characteristics associated with various soil types and SOC levels and is also utilized in this study. A soil spectral library is a comprehensive collection of soil reflectance spectra, which are measurements of how soils reflect electromagnetic radiation across different wavelengths, typically in the visible, near-infrared (NIR), and mid-infrared (MIR) regions of the spectrum. SSL provides the information on spectral characteristics associated with various soil types and for various depths. The SSL data integration is performed using Python, NumPy, and Pandas. SSL data preprocessing involved feature extraction utilizing spectral profiles and associated SOC levels. The primary goal of forming distinctive zones is to strategically guide the collection of soil samples for the precise estimation of the SOC. This approach integrates temporally weighted crop layers depicting cropping pattern, climatic variability, and soil properties, such as SOC and bulk density, to craft zones that shows unique characteristics influencing SOC dynamics. These zones are used to select some sample plots to collect the data on actual SOC, SSL, management practices and satellite datasets. Actual SOC data is garnered through collaborative efforts with local agricultural bodies and soil survey agencies, employing field-based sampling campaigns and laboratory analyses to ensure representative samples across depths. At step 404 of the method 400, the one or more hardware processors 104 are configured to integrate the plurality of spectral embeddings, the plurality of temporal embeddings, and the SSL attention score into a joint feature space to obtain a domain-aware machine learning (ML) model 110A. The embedding layers for satellite-derived spectral bands or indices generated using optical and SAR data is to transform high-dimensional spectral information into a dense vector space. This transformation captures inherent relationships between different spectral features, allowing the subsequent layers of the neural network to efficiently extract and learn relevant patterns. This helps in the reduction of dimensionality, aiding the model in discerning important spectral characteristics while mitigating the curse of dimensionality. Let Xspectral represent the input spectral data, and the embedding layer transforms it into a dense vector space E(Xspectral). Mathematically, "E" signifies the embedding function. Further, the management practices data is utilized into the model based on the proxies derived from satellite and weather datasets. Moreover, these proxies are associated with temporal aspects or the time domain. The incorporation of temporal embeddings for management proxies is essential to capture the temporal variations in agricultural practices. By leveraging recurrent neural network (RNN) layers or temporal embeddings, the model can effectively encode the temporal dynamics of management practices, enhancing its ability to adapt to changes over time. This contributes to improved model flexibility and adaptability to evolving agricultural scenarios. The recurrent neural network (RNN) layer or temporal embeddings are generated to capture temporal variations. Let Xproxy represent the management proxies, and Etemporal(Xproxy) symbolize the temporal embedding function. The attention score generated by the SSL is to dynamically focus on relevant spectral signatures from the SSL. This mechanism allows the model to assign varying levels of importance to different SSL features, ensuring that the model emphasizes the most informative components. This helps the model to adapt its attention to different soil characteristics, enhancing overall performance. Let $X_{SSL}$ denote SSL features, and $A(X_{SSL})$ represent the attention mechanism, dynamically focusing on relevant spectral features. Further, the spectral embedding, the temporal embeddings of the management proxies, and SSL attention scores are integrated into a joint feature space. Integrating embedded spectral features, temporal embeddings, and SSL attention scores into a joint feature space ensures that the model comprehensively considers multiple sources of information. The advantage of joint feature fusion is the holistic representation of diverse data types, capturing synergies and dependencies between spectral, temporal, and library-based features. This contributes to a more informed and context-aware SOC estimation.

The joint feature vector is denoted as:

$$X_{joint} = [E(X_{spectral}), E_{temporal}(X_{proxy}), A(X_{SSL})] \qquad (1)$$

*Wherein,*

Xjoint : This represents the joint input feature vector used in the domain-aware machine learning model.
E(Xspectral ): This term represents the embedding of spectral features extracted from satellite data. It transforms spectral information into a dense vector space capturing relationships between different spectral features.

*Etemporal* (*Xproxy*): This term represents the temporal embedding of management proxies. It encapsulates temporal variations in management practices over time using recurrent neural network (RNN) layers or temporal embeddings.

*A*(*XSSL*): This term represents the attention mechanism applied to features derived from the Soil Spectral Library (SSL). It dynamically focuses on relevant spectral features for SOC estimation, acknowledging the influence of SSL-based information.

At step 406 of the method 400, the one or more hardware processors 104 are configured to apply a loss function to the domain-aware ML model 110A. The loss function employs the plurality of temporal embeddings and the SSL signatures to minimize an error in the SOC prediction for the ROI by the domain-aware ML model 110A. The loss function is devised that minimizes the error in the SOC prediction while incorporating SSL-based information and management proxies. The devised loss function, which incorporates SSL-based information and management proxies, serves to guide the model during training. By minimizing this comprehensive loss, the model is incentivized to not only predict SOC accurately but also consider the domain-specific features. The advantage is a more robust and context-aware model, capable of handling diverse input information for precise SOC estimation. This step provides the improved estimates of surface SOC.

The overall loss function is denoted as:

$$L(Y_{true}, f_{NN}(X_{joint}; \theta)) \qquad (2)$$

This represents the loss function used to measure the discrepancy between the true labels $Y_{true}$ and the predictions of the neural network model $f_{NN}(X_{joint}; \theta)$

*Ytrue* : True labels or ground truth values.

*fNN* (*Xjoint* ;$\theta$): Predictions of the neural network model *fNN* given the input data *Xjoint* and the model parameters $\theta$

$\theta$: Model parameters, including weights and biases.

At step 408 of the method 400, the one or more hardware processors 104 are configured to generate a temporal SOC for the ROI by a process-based model 110B. The process-based model 110B utilizes a plurality of factors affecting a historical surface SOC and a historical vertical SOC over a period of time to predict the temporal SOC. The temporal SOC is an estimate of SOC with respect to spatial-temporal variability of soil attributes over a period of time. The variation in soil attributes is a result of the interaction of natural and human factors, which can be largely attributed to climate, terrain, parent material, time and biology. The temporal SOC estimation involves analyzing the historical surface SOC, and the historical vertical SOC gathered over a period of time.

For the comprehensive understanding of the SOC dynamics within selected plots from each designated zone or cluster, the process-based models such as Century, DayCent, HOLOS, DNDC are employed. While estimating the temporal SOC, essential datasets are gathered having a chronological record of soil properties, climate data, land management practices, and initial SOC concentrations within the chosen plots. These datasets serve as the foundational input for the simulation. Subsequently, the process-based model 110B is selected based on suitability for the specific region or ecosystem. The simulation of the temporal SOC based on the chronological record involves the dynamic representation of SOC turnover processes, considering factors such as microbial activity, plant-soil interactions, and environmental conditions. The predictive capability of the model 110 is harnessed to generate temporal SOC profile. The temporal SOC profile depicts varying SOC concentrations in response to varying inputs and environmental scenarios. The temporal profiling offers insights into the impacts of land management practices, climate variations, and soil properties on SOC dynamics over time. Upon completion of the simulations, the outcomes provide a detailed understanding of SOC patterns fluctuating seasonally and annually within the selected plots. These insights contribute to get the insights on the complex interplay of biological, chemical, and physical processes influencing SOC dynamics.

At step 410 of the method 400, the one or more hardware processors 104 are configured to obtain a correction factor by subtracting the temporal SOC predicted by the process-based model 110B and the surface SOC estimated from the domain-aware ML model 110A. For the ROI, the temporal SOC (i.e. the actual surface SOC) simulated by the process-based model 110B serves as the target variable. The domain-aware model 110A is then trained, with temporal SOC estimates from the process-based model 110B, alongside spectral data from the plots and satellite datasets, serving as features. The trained domain-aware ML model 110A aims to predict the surface SOC, drawing insights from both empirical and spectral information. Subsequently, the average difference between the temporal SOC values derived from the process-based model 110B and the predicted values from the domain-aware ML model 110A is computed. This difference acts as the correction factor, capturing the nuanced discrepancies between the two modeling approaches. Importantly, this correction factor is then applied to the surface SOC estimates from the domain-aware ML model 110A, serving as an adjustment mechanism to align the ML-based estimations with the dynamics simulated by the process-based model 110B.

The surface SOC is represented as:

$$SSOC_{Process} = f(\ SSOC_{ML},\ spectral\ data,\ satellite\ data) \quad (3)$$

Where $SSOC_{ML}$ is the surface SOC derived from the domain-aware ML model 110A and $SSOC_{Process}$ is the surface SOC derived from the process-based model 110B, then the correction factor is represented by:

$$CF_{Surface} = Average\ (SSOC_{ProcessPredicted} - SSOC_{ProcessActual})\ (4)$$

Where,

$CF_{Surface}$ represents the correction factor for improved SOC estimates

$SSOC_{ProcessPredicted}$ represents the predicted SSOC using the ML model

$SSOC_{ProcessActual}$ represents the actual SSOC from the process-based model

At step 412 of the method 400, the one or more hardware processors 104 are configured to estimate a corrected surface SOC by applying the correction factor to the surface SOC estimates obtained from the domain-aware ML model 110A. This step considers the surface SOC ($SSOC_{ML}$) derived from the domain-aware ML model 110A along with correction factor ($CF_{Surface}$) derived in above step. The corrected surface SOC is represented by:

$$SSOC_{corrected} = SSOC_{ML} + CF_{Surface} \quad\quad (5)$$

Where, $SSOC_{corrected}$ is the corrected surface SOC

At step 414 of the method 400, the one or more hardware processors 104 are configured to obtain a plurality of depth specific SOC fractions in the ROI. The plurality of depth specific SOC fractions are calculated by first estimating a static vertical SOC profile at a plurality of depths from the global soil information system. In an embodiment, the global soil information system is SoilGrids™. The data from SoilGrids™ includes various soil properties including SOC at the plurality of depth intervals. The data is available from 0-200 cm at different depths. The dataset is static which means it has been generated once using the environmental co-variates such as climate vegetation and remote sensing data. This serves as a baseline vertical SOC profile. The SOC obtained from SoilGrids™ at depth $d_1$ is represented as SoilGrids$_{SOCd1}$. Then, a temporally weighted SOC profile is generated by the process-based model 110B wherein the process-based model 110B calculates the temporal SOC at the plurality of depths. Further, the plurality of temporally weighted SOC fractions obtained from the process-based models are estimated. For each zone of the ROI derived initially, the process-based model simulates the temporal SOC and the vertical SOC. This data is utilized to estimate the temporally weighted SOC profile for each zone. Here say, for time instance $t_1$, $t_2$, ....$t_n$, the SOC at depth $d_1$ is SOC$_{process(d1)(t1)}$, SOC$_{process(d1)(t2)}$, SOC$_{process(d1)(tn)}$ and temporally weighted SOC from the process-based models at depth d1 is SOC$_{process(d1)(temp)}$.

Next, SOC fractions are estimated at the plurality of depths from the SSL wherein the one or more SSL data reveals the SOC fractions based on general trends observed in the SOC distribution within the soil profile. Further the static vertical SOC fraction derived from the global soil information system, the temporally weighted SOC derived from the process-based model 110B, and the SOC fractions obtained from SSL are combined to obtain the depth specific SOC fractions represented by an equation:

$$SOCFractiond1 = (w1* SOC_{process(d1)(temp)}) + (w2 * SoilGridsSOCd1) + (w3 * SOC_{SSL(d1)}) \quad\quad (6)$$

Where,

SOCFractiond1 represents the fraction of SOC at depth *d1*,

$w1, w2, w3$ represents the weights assigned to each component in the calculation of the SOC fraction at depth *d1*. They represent the relative importance or contribution of each component,

SOC$_{process(d1)(temp)}$ represents the SOC proportion at depth *d1* derived from the process-based models, considering the temporal dynamics (temp) of SOC,

SoilGridsSOC*d*1 represents the SOC proportion at depth *d1* obtained from SoilGrids™ data, which provides global gridded soil information, and

SOC$_{SSL(d1)}$ represents the SOC proportion at depth *d1* derived from the SSL that contains spectral profiles associated with various soil properties and SOC levels.

Next, the plurality of SOC fractions are derived from the SSL. The SSL data encompass soil properties such as soil texture and soil type information and is utilized to estimate the plurality of fractions of the SOC at various depths relative to surface SOC for a given pixel/point. Initially, soil texture and type characteristics are used to characterize the soil profile, providing insights into SOC distribution patterns. Integration of the SSL data, including spectral profiles and associated SOC values at different depths enables extraction of relevant spectral features associated with the SOC variations. An algorithm or heuristic approach is then deployed to estimate SOC fractions based on empirical rules or relationships derived from the SSL dataset, capturing general trends observed in the SOC distribution within the soil profile. Here say, SSL based SOC fraction at depth $d_1$ is $SOC_{SSL(d1)}$.

Finally the temporally weighted fractions derived from the process-based models, static fractions derived from the SoilGrids™ data and fractions using the SSL are combined and weighted average is estimated to derive the final depth specific fractions such as:

$$SOC_{Fractiond1} = (w1 * SOC_{process(d1)(temp)}) + (w2 * SoilGridsSOC_{d1}) + (w3 * SOC_{SSL(d1)}) \tag{7}$$

At step 414 of the method 400, the one or more hardware processors 104 are configured to estimate a vertically distributed SOC for the ROI by combining the corrected surface SOC and depth specific SOC fraction at the ROI. The estimation of the dynamic SOC at depth $d_1$ using the corrected surface SOC estimates and the fraction at depth $d_1$ is represented as:

$$SOC_{d1(corrected)} = (SSOC_{corrected} * SOCFraction_{d1}) + SSOC_{corrected} \tag{8}$$

where

$SOC_{d1(corrected)}$ represents the improved estimate of SOC at depth $d1$,
$SSOC_{corrected}$ represents the improved estimate of surface soil organic carbon (SSOC), and
$SOCFraction_{d1}$ represents the fraction of SOC at depth.

Therefore, the weighted average of the SOC fractions derived from the process-based model, depth informed spectral embeddings from the SSL and SoilGrids™ data is estimated to get the final SOC fractions at each depth. The vertical SOC estimation model 110 acquires the SOC fractions at each depth of the ROI and the corrected surface SOC from the domain-aware ML model 110A. The vertical SOC estimation model 110 processes the SOC fractions at each depth and the corrected surface SOC to estimate vertical profile of SOC at each pixel. This dynamically estimated vertical distribution of SOC is based on inferences of the surface SOC, temporal SOC, SOCs from SSL and other global soil information system while systematically processing the SOCs collected from these source through a combined approach utilizing the domain-aware ML-model 110A and the process-based model 110B. The present invention discloses a robust framework utilizing inferences generated from the process-based model 110B to train the domain-aware ML model 110A to dynamically estimating vertical SOC profile.

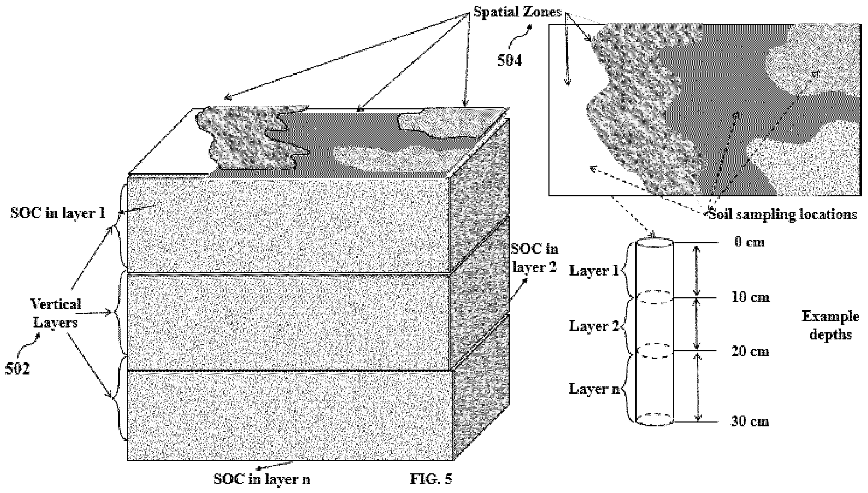

FIG. 5 illustrates agricultural land division into a plurality of vertical layers 502 and a plurality of spatial zones 504 to estimate SOC in a region of interest (ROI), according to some embodiments of the present disclosure. While dynamic estimation of vertical SOC, the soil is the region of interest is divided into the plurality of vertical layers 502 and the plurality of spatial zones 504. The underground soil is divided equally into the plurality of vertical layers 502 as d1, d2, d3...... dn to cover the vertical depth up to which SOC can be estimated. Exemplary depths are represented as d1 having a depth of 0-10 centimeters (cm), d2 having a dept of 10-20 centimeters (cm), and dn having a dept of 20-30 centimeters (cm. The plurality of spatial zones 504 are formed based on the similarity in SOC and can be used to collect representative SOC ground samples, which may be used for modelling. While estimated vertical SOC for the region of interest, the area is divided into the plurality of vertical layers 502 and the plurality of spatial zones 504 for the calculation involved in modeling vertical SOC as per the method disclosed in the present invention.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

The embodiments of the present disclosure herein addresses unresolved problem of dynamic estimation of vertical SOC by combined analysis of the domain-aware ML model and the process-based model. The domain-aware data utilizes holistic framework of soil by considering spectral embeddings from satellite data, temporal embeddings from management practices and relevant information from SSL. The process-based model contributes to improved SOC estimation as well as processes temporally weighted depth-wise SOC fractions. A static vertical SOC fraction is also obtained from the global soil information system. Finally, vertically distributed SOC for the ROI is estimated by combining the corrected surface SOC and depth specific SOC fraction at the ROI. The invention provide accurate estimation of vertical SOC from a decent depth level ranging from 0 cm-300 cm. The SOC estimation from the disclosed method provides valuable insights into land-use changes, deforestation, and human-induced alterations, supporting sustainable land management and biodiversity conservation.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1.   A processor implemented method (400), comprising:

   receiving (402), via one or more hardware processors, (i) a plurality of spectral embeddings derived from a satellite data, (ii) a plurality of temporal embeddings derived from a plurality of management proxies, and (iii) an attention score from a soil spectral library (SSL) based on one or more soil spectral signatures derived from the SSL for a region of interest (ROI);
   integrating(404), via the one or more hardware processors, the plurality of spectral embeddings, the plurality of temporal embeddings, and the SSL attention score into a joint feature space to obtain a domain-aware machine learning (ML) model;

applying (406), via the one or more hardware processors, a loss function to the domain-aware ML model, wherein the loss function employs the plurality of temporal embeddings and the one or more soil spectral signatures to minimize an error in a surface soil organic carbon (SOC) prediction for the ROI;

generating (408), via the one or more hardware processors, a temporal SOC for the ROI by a process-based model, wherein the process-based model utilizes the plurality of spectral embeddings, the plurality of temporal embeddings and the SSL data to analyze a historical surface SOC and a historical vertical SOC over a period to generate the temporal SOC;

obtaining (410), via the one or more hardware processors, a correction factor by subtracting the temporal SOC generated by the process-based model and the surface SOC estimated from the domain-aware ML model;

estimating (412), via the one or more hardware processors, a corrected surface SOC by applying the correction factor to the surface SOC estimates obtained from the domain-aware ML model;

obtaining (414), via the one or more hardware processors, one or more depth specific SOC fractions of the ROI, by:

receiving a static vertical SOC profile at a plurality of depths from a global soil information system;

generating a temporally weighted SOC profile by the process-based model, wherein the process-based model calculates the temporal SOC at the plurality of depths;

estimating a plurality of SOC fractions at the plurality of depths from the SSL, wherein the plurality of SOC fractions represents one or more general trends of one or more soil properties in the ROI; and

combining the static vertical SOC fraction received from the global soil information system, the temporally weighted SOC derived from the process-based model, and the plurality of SOC fractions estimated from the SSL to obtain the

one or more depth specific SOC fractions; and

estimating (416), via the one or more hardware processors, a vertically distributed SOC for the ROI by combining the corrected surface SOC of the domain-aware ML model and the one or more depth specific SOC fractions of the ROI.

2. The method as claimed in claim 1, wherein, (a) the plurality of spectral embeddings transforms a high-dimensional spectral information received from the satellite data into a dense vector space to enable subsequent layers of the domain-aware ML model to efficiently extract and learn relevant patterns, (b) the plurality of temporal embeddings capture temporal dynamics of the plurality of management proxies to enable the domain-aware ML model to adapt to evolving agricultural scenarios, and (c) the soil spectral signatures are utilized to embed an attention mechanism in the domain-aware ML model, wherein the attention mechanism trains the domain-aware ML model to adapt to one or more characteristic variations among soil and generate the attention score.

3. The method as claimed in claim 1, wherein the loss function embeds a plurality of domain-specific features to the domain-aware ML model to enhance the surface SOC prediction accuracy of the domain-aware ML model by incorporating the plurality of the temporal embeddings and the soil spectral signatures, and wherein the loss function is denoted as:

$$L(Y_{\text{true}}, f_{\text{NN}}(X_{\text{joint}}; \theta)),$$

where,

L is the loss function measuring discrepancy between one or more true labels $Y_{\text{true}}$ and one or more predictions of the domain-aware ML model $f_{\text{NN}}(X_{\text{joint}}; \theta)$, $Y_{true}$ represents the one or more true labels or ground truth values, $f_{NN}(X_{joins}; \theta)$ represents the one or more predictions of the domain-aware ML model $f_{NN}$ given the input data $X_{joint}$ and the model parameters $\theta$, and

$\theta$ represents one or more model parameters comprising a plurality of weights and a plurality of biases.

4. The method as claimed in claim 1, wherein the temporal SOC estimations utilizes a plurality of insights about an impact of one or more land management practices, one or more climatic variations, and the plurality of soil properties on the SOC dynamics over time to identify a pattern of the SOC based on at least one of a seasonal fluctuation and an annual fluctuation.

5. The method as claimed in claim 1, wherein the vertical distribution of SOC at a desired depth $d_1$ is expressed as:

$$SOC_{d1(corrected)} = (SSOC_{corrected} * SOCFraction_{d1}) + SSOC_{corrected}$$

where, $SOC_{d1(corrected)}$ represents the improved SOC at depth $d_1$,
$SSOC_{corrected}$ represents the corrected surface SOC (SSOC), and
$SOC_{Fractiond1}$ represents fraction of SOC at depth $d1$.

6. A system (100), comprising:

a memory (102) storing instructions;
one or more communication interfaces (106); and
one or more hardware processors (104) coupled to the memory (102) via the one or more communication interfaces (106), wherein the one or more hardware processors (104) are configured by the instructions to:

receive (i) a plurality of spectral embeddings derived from a satellite data, (ii) a plurality of temporal embeddings derived from a plurality of management proxies, and (iii) an attention score from a soil spectral library (SSL) based on one or more soil spectral signatures derived from the SSL for a region of interest (ROI);
integrate the plurality of spectral embeddings, the plurality of temporal embeddings, and the SSL attention score into a joint feature space to obtain a domain-aware machine learning (ML) model;
apply a loss function to the domain-aware ML model, wherein the loss function employs the plurality of temporal embeddings and the one or more soil spectral signatures to minimize an error in a surface soil organic carbon (SOC) prediction for the ROI;
generate a temporal SOC for the ROI by a process-based model, wherein the process-based model utilizes the plurality of spectral embeddings, the plurality of temporal embeddings and the SSL data to analyze a historical surface SOC and a historical vertical SOC over a period to generate the temporal SOC;
obtain a correction factor by subtracting the temporal SOC generated by the process-based model and the surface SOC estimated from the domain-aware ML model;
estimate a corrected surface SOC by applying the correction factor to the surface SOC estimates obtained from the domain-aware ML model;
obtain one or more depth specific SOC fractions of the ROI, by:

receiving a static vertical SOC profile at a plurality of depths from a global soil information system;
generating a temporally weighted SOC profile by the process-based model, wherein the process-based model calculates the temporal SOC at the plurality of depths;
estimating a plurality of SOC fractions at the plurality of depths from the SSL, wherein the plurality of SOC fractions represents one or more general trends of one or more soil properties in the ROI; and
combining the static vertical SOC fraction received from the global soil information system, the temporally weighted SOC derived from the process-based model, and the plurality of SOC fractions estimated from the SSL to obtain the

one or more depth specific SOC fractions; and

estimate a vertically distributed SOC for the ROI by combining the corrected surface SOC of the domain-aware ML model and the one or more depth specific SOC fractions of the ROI.

7. The system as claimed in claim 6, wherein, (a) the plurality of spectral embeddings transforms a high-dimensional spectral information received from the satellite data into a dense vector space to enable subsequent layers of the domain-aware ML model to efficiently extract and learn relevant patterns, (b) the plurality of temporal embeddings capture temporal dynamics of the plurality of management proxies to enable the domain-aware ML model to adapt to evolving agricultural scenarios, and (c) the soil spectral signatures are utilized to embed an attention mechanism in the domain-aware ML model, wherein the attention mechanism trains the domain-aware ML model to adapt to one or more characteristic variations among soil and generate the attention score.

8. The system as claimed in claim 6, wherein the loss function embeds a plurality of domain-specific features to the domain-aware ML model to enhance the surface SOC prediction accuracy of the domain-aware ML model by incorporating the plurality of the temporal embeddings and the soil spectral signatures, and wherein the loss function is

denoted as:

$$L(Y_{true}, f_{NN}(X_{joint}; \theta)),$$

where,

L is the loss function measuring discrepancy between one or more true labels $Y_{true}$ and one or more predictions of the domain-aware ML model $f_{NN}(X_{joint}; \theta)$,

$Y_{true}$ represents the one or more true labels or ground truth values,

$f_{NN}(X_{joint}; \theta)$ represents the one or more predictions of the domain-aware ML model $f_{NN}$ given the input data $X_{joint}$ and the model parameters $\theta$, and

$\theta$ represents one or more model parameters comprising a plurality of weights and a plurality of biases.

9. The system as claimed in claim 6, wherein the temporal SOC estimations utilizes a plurality of insights about an impact of one or more land management practices, one or more climatic variations, and the plurality of soil properties on the SOC dynamics over time to identify a pattern of the SOC based on at least one of a seasonal fluctuation and an annual fluctuation.

10. The system as claimed in claim 6, wherein the vertical distribution of SOC at a desired depth $d_1$ is expressed as:

$$SOC_{d1(corrected)} = (SSOC_{corrected} * SOCFraction_{d1}) + SSOC_{corrected}$$

where, $SOC_{d1(corrected)}$ represents the improved SOC at depth $d_1$,

$SSOC_{corrected}$ represents the corrected surface SOC (SSOC), and

$SOC_{Fractiond1}$ represents fraction of SOC at depth $d1$.

11. One or more non-transitory machine-readable information storage mediums comprising one or more instructions which when executed by one or more hardware processors cause:

receiving (i) a plurality of spectral embeddings derived from a satellite data, (ii) a plurality of temporal embeddings derived from a plurality of management proxies, and (iii) an attention score from a soil spectral library (SSL) based on one or more soil spectral signatures derived from the SSL for a region of interest (ROI);

integrating the plurality of spectral embeddings, the plurality of temporal embeddings, and the SSL attention score into a joint feature space to obtain a domain-aware machine learning (ML) model;

applying a loss function to the domain-aware ML model, wherein the loss function employs the plurality of temporal embeddings and the one or more soil spectral signatures to minimize an error in a surface soil organic carbon (SOC) prediction for the ROI;

generating a temporal SOC for the ROI by a process-based model, wherein the process-based model utilizes the plurality of spectral embeddings, the plurality of temporal embeddings and the SSL data to analyze a historical surface SOC and a historical vertical SOC over a period to generate the temporal SOC;

obtaining a correction factor by subtracting the temporal SOC generated by the process-based model and the surface SOC estimated from the domain-aware ML model;

estimating a corrected surface SOC by applying the correction factor to the surface SOC estimates obtained from the domain-aware ML model; obtaining (414), via the one or more hardware processors, one or more depth specific SOC fractions of the ROI, by:

receiving a static vertical SOC profile at a plurality of depths from a global soil information system;

generating a temporally weighted SOC profile by the process-based model, wherein the process-based model calculates the temporal SOC at the plurality of depths;

estimating a plurality of SOC fractions at the plurality of depths from the SSL, wherein the plurality of SOC fractions represents one or more general trends of one or more soil properties in the ROI; and

combining the static vertical SOC fraction received from the global soil information system, the temporally weighted SOC derived from the process-based model, and the plurality of SOC fractions estimated from the SSL to obtain the one or more depth specific SOC fractions; and

estimating a vertically distributed SOC for the ROI by combining the corrected surface SOC of the domain-aware ML model and the one or more depth specific SOC fractions of the ROI.

12. The one or more non-transitory machine-readable information storage mediums as claimed in claim 11, wherein (a) the plurality of spectral embeddings transforms a high-dimensional spectral information received from the satellite data into a dense vector space to enable subsequent layers of the domain-aware ML model to efficiently extract and learn relevant patterns, (b) the plurality of temporal embeddings capture temporal dynamics of the plurality of management proxies to enable the domain-aware ML model to adapt to evolving agricultural scenarios, and (c) the soil spectral signatures are utilized to embed an attention mechanism in the domain-aware ML model, wherein the attention mechanism trains the domain-aware ML model to adapt to one or more characteristic variations among soil and generate the attention score.

13. The one or more non-transitory machine-readable information storage mediums as claimed in claim 11, wherein the loss function embeds a plurality of domain-specific features to the domain-aware ML model to enhance the surface SOC prediction accuracy of the domain-aware ML model by incorporating the plurality of the temporal embeddings and the soil spectral signatures, and wherein the loss function is denoted as:

$$L(Y_{true}, f_{NN}(X_{joint}; \theta)),$$

where,

L is the loss function measuring discrepancy between one or more true labels $Y_{true}$ and one or more predictions of the domain-aware ML model $f_{NN}(X_{joint}; \theta)$, $Y_{true}$ represents the one or more true labels or ground truth values, $f_{NN}(X_{joint}; \theta)$ represents the one or more predictions of the domain-aware ML model $f_{NN}$ given the input data $X_{joint}$ and the model parameters $\theta$, and
$\theta$ represents one or more model parameters comprising a plurality of weights and a plurality of biases.

14. The one or more non-transitory machine-readable information storage mediums as claimed in claim 11, wherein the temporal SOC estimations utilizes a plurality of insights about an impact of one or more land management practices, one or more climatic variations, and the plurality of soil properties on the SOC dynamics over time to identify a pattern of the SOC based on at least one of a seasonal fluctuation and an annual fluctuation.

15. The one or more non-transitory machine-readable information storage mediums as claimed in claim 11, wherein the vertical distribution of SOC at a desired depth $d_1$ is expressed as:

$$SOC_{d1(corrected)} = (SSOC_{corrected} * SOCFraction_{d1}) + SSOC_{corrected}$$

where, $SOC_{d1(corrected)}$ represents the improved SOC at depth $d_1$,
$SSOC_{corrected}$ represents the corrected surface SOC (SSOC), and
$SOC_{Fractiond1}$ represents fraction of SOC at depth $d1$.

## System 100

| Processors(s) 104 | I/O Interface(s) 106 |
|---|---|

### Database 108

### Cloud server 116

### Memory 102

#### VERTCAL SOC ESTIMATION MODEL 110

Domain aware ML model 110A

Process-based model 110B

SSL data processing module 112A

GSI data processing module 112B

FIG. 1

FIG. 2

FIG. 3

receiving (a) a plurality of spectral embeddings from satellite data, (b) a plurality of temporal embeddings from management proxies derived from satellite data, (c) soil spectral library (SSL) attention score based on soil spectral signatures derived from the SSL for a region of interest (ROI)

402

integrating the plurality of spectral embeddings, the plurality of temporal embeddings, and the SSL attention score into a joint feature space to obtain a domain-aware ML model

404

applying a loss function to the domain-aware ML model wherein the loss function minimizes an error in the SOC prediction while incorporating the plurality of temporal embeddings, and the soil spectral signatures predicting surface SOC for the ROI using the domain-aware ML model

406

generating temporal SOC for the ROI by a process-based model wherein the process-based model utilizes a plurality of factors affecting historical surface SOC and historical vertical SOC over a period to predict the temporal SOC

408

A

FIG. 4A

EP 4 668 197 A1

400

(A)

obtaining a correction factor by subtracting temporal SOC predicted by the process-based model and the surface SOC estimated from the domain-aware ML model 〜 410

estimating an improved surface SOC by applying the correction factor to the surface SOC estimates obtained from the domain-aware ML model 〜 412

obtaining depth specific SOC fractions of the ROI 〜 414

estimating vertically distributed SOC for a region of interest by combining improved surface SOC and depth specific SOC fraction at the ROI 〜 416

FIG. 4B

Spatial Zones

504

SOC in layer 1

SOC in layer 2

Vertical Layers

502

SOC in layer n

Soil sampling locations

0 cm

Layer 1

10 cm

Layer 2

20 cm

Layer n

30 cm

Example depths

FIG. 5

**EUROPEAN SEARCH REPORT**

Europäisches Patentamt
European Patent Office
Office européen des brevets

| Application Number |
| --- |
| EP 25 18 3058 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
| --- | --- | --- | --- |
| A | VAUDOUR EMMANUELLE ET AL: "Satellite Imagery to Map Topsoil Organic Carbon Content over Cultivated Areas: An Overview", REMOTE SENSING, vol. 14, no. 2917, 18 June 2022 (2022-06-18), XP055946696, DOI: 10.3390/rs14122917 * abstract * * sections 1-5 * * figures 1-7 * | 1-15 | INV. G06Q50/02 G01N33/24 G06N3/08 A01B79/00 |
| A | MOSTAFA EMADI ET AL: "Predicting and Mapping of Soil Organic Carbon Using Machine Learning Algorithms in Northern Iran", ARXIV.ORG, CORNELL UNIVERSITY LIBRARY, 201 OLIN LIBRARY CORNELL UNIVERSITY ITHACA, NY 14853, 12 July 2020 (2020-07-12), XP081726716, DOI: 10.3390/RS12142234 * abstract * * sections 1-4 * * figures 1-9 * | 1-15 | |

TECHNICAL FIELDS SEARCHED (IPC)

G06Q
G01N
G06N
A01B

The present search report has been drawn up for all claims

| Place of search | Date of completion of the search | Examiner |
| --- | --- | --- |
| Munich | 3 November 2025 | Körbler, Günther |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202421046889